# EUROPEAN PATENT SPECIFICATION

(11) **EP 1 916 013 B1**
(45) Date of publication and mention of the grant of the patent: **15.09.2010**
(21) Application number: 07020933.3
(22) Date of filing: 25.10.2007
(51) Int. Cl.: A61N 1/30, A61N 1/32

(54) **Hyperthermia device for the selective treatment and monitoring of surface tissue**
Hyperthermievorrichtung zur selektiven Behandlung und Kontrolle von Oberflächengewebe
Dispositif d'hyperthermie pour le traitement sélectif et la surveillance des tissus en surface

(30) Priority: 25.10.2006 DE 102006050369
(43) Date of publication of application: 30.04.2008
(73) Proprietor: Oncotherm Kft., 2071 Páty (HU)
(72) Inventor: Szasz, Andras, 2071 Páty (HU); Szasz, Oliver, 2071 Páty (HU)
(74) Representative: Arth, Hans-Lothar

(56) References cited:
- EP-A- 0 587 269
- WO-A-00/67837
- WO-A-01/00274
- WO-A-97/02861
- WO-A-97/36645
- WO-A-97/40886
- US-A- 4 164 226
- US-A1- 2004 019 371
- US-B1- 6 609 032

## Description

The present invention relates to an inventive electric field capacitive coupled energy transfer means for selective treatment of surface tissue. The improved device selectively delivers energy and heat to surface tissues of mammals.

### Background of the invention

Hyperthermia devices can be used to force energy absorption in tissue to cause damage to unwanted structures and/or increase the temperature of the targeted area above the normal body temperature. One use of hyperthermia devices is the treatment of cancer. In the case of cancer treatment, tumour cells are more sensitive to stresses, for example, heat and/or electromagnetic fields and chemical treatments, than the surrounding normal tissue cells. Therefore the aim is to pump sufficient energy to the tumour tissue to irreversibly damage the tumour cells, but which can be tolerated by normal tissue cells.

Conventional non-invasive electrical field hyperthermia devices comprise a single active electrode and a counter electrode that are placed on opposing sides of the target tissue to be heated. Although energy is also delivered to surface tissue by the active electrode, this arrangement is optimised to deliver energy to an internal tissue target site, such as an internal tumour, and the delivery of energy to surface tissue is considered an undesirable side effect.

To use electrical field hyperthermia to treat surface tissue diseases and conditions, such as articular and joint conditions, skin cancer, and surface blood circulation, a hyperthermia device that selectively delivers energy to target surface tissues without affecting internal tissue is required, as delivery of energy to internal non-target tissue when treating surface tissue can cause the unwanted side effect of damaging non-target internal tissues.

Herein, we report the development of an electric field capacitive coupled energy transfer means and method for selectively delivering energy to surface tissue, for selectively heating surface tissue and for selectively monitoring the electrical properties of surface tissue. This is achieved by providing an inventive arrangement of multiple positive and negative electrodes in the same plane on an electric field coupled energy transfer means. The inventive electric field capacitive coupled energy transfer means and method can be used to treat and monitor all types of surface tissue diseases and conditions.

The prior art does not disclose an electric field capacitive coupled hyperthermia device that selectively delivers energy and heat selectively to surface tissues. Instead the prior art hyperthermia devices only heat surface tissues as an unwanted side effect when heating target internal tissues.

EP 0 251 746 discloses typical examples of prior art hyperthermia devices for external or internal use, where an active electrode and a counter electrode are situated on opposing sides of a target tissue.

US 6,330,479 discloses a microwave hyperthermia applicator in the form of an microwave antenna array for the radiative heat treatment of tissue. This dipole-arrangement works like a radiative antenna, characterized by a Poynting vector (proportional to the vectorial product of electric and magnetic fields), and is effective at high RF-frequencies (more than 100 MHz).

Gelvich and Mazokhin (Contact flexible microstrip applicators (CFMA) in a range from microwave up to short waves IEEE Transactions on Biomedical Engineering Vol. 49 (9), pp. 1015 1023, 2002) disclose a flexible microstrip applicator in the form of a dipole antenna for the radiative heat treatment of tissue. This applicator has both dipole and capacitor antennas, and cannot be used at frequencies lower than 27 MHz. The capacitive applicator can only be in the form of a strip because the bent cylinder axis must be parallel with the electric field vector.

EP 0 207 739 discloses device comprising microwave applicators for radiating electromagnetic energy to local regions of body tissue. The microwave applicators are for internal use and are inserted into the tissue to be treated. The microwave applicators can also comprise a separate temperature sensing apparatus.

US 4,164,226 discloses iontophoretic galvanic coupled treatment electrode for use in direct contact with the skin. The treatment electrode can comprise intermingled positive and negative electrodes. The treatment electrode is for use in treating dermatological and cosmetic problems.

EP 0 970 719 discloses a device having alternating positive and negative galvanic coupled electrode structures which have an electrolyte layer laminated to the electrode, where the electrodes are for use in direct contact with the skin. The positive electrode structure can comprise multiple positive electrodes and the negative electrode structure can comprise multiple negative electrodes. The device is proposed for use in iontophoresis or in diathermy knives.

GB 2 281 863 discloses a device having multiple galvanic coupled electrodes which can have different polarities and can deliver high frequency electric current. The electrodes are for use in direct contact with the skin and are for use in diathermy knives.

Document WO 00/67837 discloses a method of electroperforation enhanced delivery of active agents which uses direct current.

Although intermingled positive and negative electrodes have previously been used in galvanic coupled electrode structures, this arrangement has not been applied to capacitive coupled electrodes. The operation of galvanic coupled electrodes requires a high electric field strength to drive the galvanic connection and a discrete switching between the positive and negative poles.

In contrast to galvanic coupled electrodes, capacitive coupled electrodes have no discrete switching between the positive and negative poles and instead cycle in a sine wave form between the positive and negative poles. Therefore there is no similarity in the operation of the capacitive and galvanic electrodes and no suggestion that an alternating positive and negative electrode arrangement would provide any advantage in a capacitive coupled energy transfer device.

Galvanic current does not flow through an ideal isolator as the material between the poles blocks the current. However alternating current apparently flows through an ideal isolator. The mechanism is the charge-division: a half-period charges one pole which acts by field only to the other pole attracting the opposite charge, while in the next half period it is reversed. The effect is due to the field, which penetrates through the ideal isolator, while the ion-flow does not. The field affects the current (called step-current) through the material. The step-current can be imagined like a domino-row falling, when no one domino runs through the line, but the effect runs through. In capacitive (alternatively changing potential) coupling after a transient period the step-current reaches a steady state and the current continuously flows through the absolute ideal insulator.

In the case of non-ideal insulators which are also not good conductors, like living tissue, the process is more complicated. Galvanic current penetrates a small distance and by adding extra ions on the surface penetrates into the skin by diffusion. In the case of capacitive coupling alternating current of radio frequency or even higher frequencies is applied. Consequently, polarity changes more frequently than 10⁷ s⁻¹. This is not useful for the normal diffusion, because it works in one half-period pro, and in the next half-period contra. In this case ionic penetration is more promoted by the step-current enhanced temperature (impedance heating) and by the changes of the dielectric-material under the step-current. In summary: galvanic coupling is a diffusion promoter and acts only on the freely moving charges, while the high-frequency current in capacitive coupling acts mainly on the fixed charges (dipoles).

It is the object of the present invention to provide an electric field capacitive coupled hyperthermia device that selectively delivers energy and heat to surface tissues and can monitor the electrical properties of surface tissues.

The object of the present invention is solved by the teaching of the independent claims. Further advantageous features, aspects and details of the invention are evident from the dependent claims, the description, and the examples of the present application.

### Description of the invention

Conventional non-invasive electrical field hyperthermia devices comprise capacitive coupling of energy, preferably with a grounded capacitor (asymmetric coupling). The single electrode having the potential change is called an active electrode. Typically the single active electrode is situated in an applicator that also comprises a transmitter bolus that couples the energy generated by the active electrode to the patient. The transmitter bolus is a typically a fluid or gas filled flexible bladder that provides good mechanical contact and excellent electromagnetic transmission with the uneven surface of the patient body, situates the active electrode at a safe distance from the patient body and delivers the energy generated by the active electrode in an even manner across the entire area where the bolus surface contacts the patient body surface.

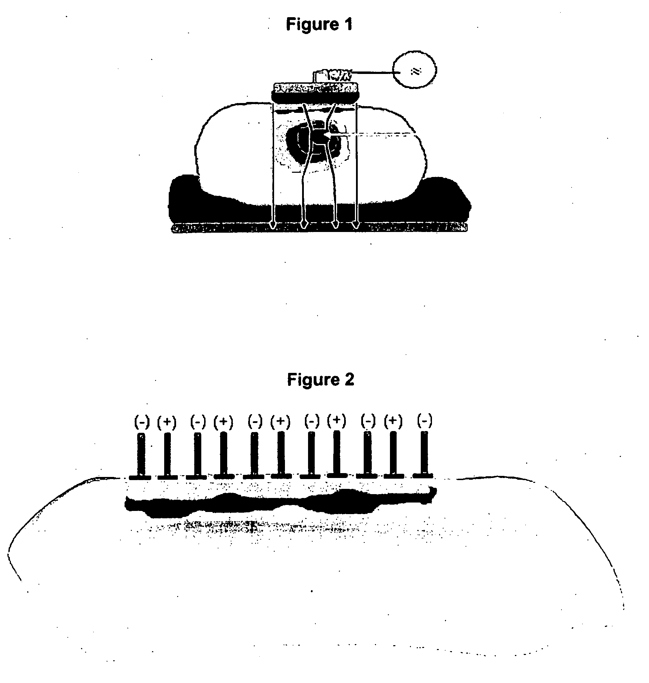

In conventional non-invasive electrical field hyperthermia devices a counter electrode (grounded electrode of the capacitor) provides an electrical field path for the energy generated by the active electrode. As shown in Figure 1 the applicator comprising the active electrode and the transmitter bolus is placed on the opposing side of the patient to the counter electrode. The desired target tissue to be treated is situated between the active electrode and the counter electrode.

Using the conventional non-invasive electrical field hyperthermia device, the energy generated by the active electrode is directed to internal tissue by the use of the counter electrode. Although surface tissue facing the active electrode also receives energy and is heated using a conventional device, the significant part of the energy is directed to the internal tissue and the delivery of energy and heating of surface tissue is considered as an undesirable side effect.

To achieve the selective treatment of only near-surface tissues the present invention provides an electric field capacitive coupled energy transfer means having a novel electrode (capacitor-antenna) arrangement for use in a hyperthermia device. The electric field coupled energy transfer means is a capacitive coupling device that forms a resonant circuit and the novel electrode arrangement results in the selective delivery of energy to only the surface tissues. The treated tissue acts as a dielectric material of the capacitor in the near-field arrangement when the distance of the tissue from the energy transfer means is less than the wavelength of the applied radio frequency (RF) wave, and the applied RF wave is tuned to fix the real-impedance value (preferably 50 Ohm). As shown in Figure 2 the novel electrode arrangement comprises multiple positive and negative electrodes provided in the same plane of an electrical field hyperthermia device applicator. The multiple positive and negative electrodes are provided in an alternating arrangement in the applicator.

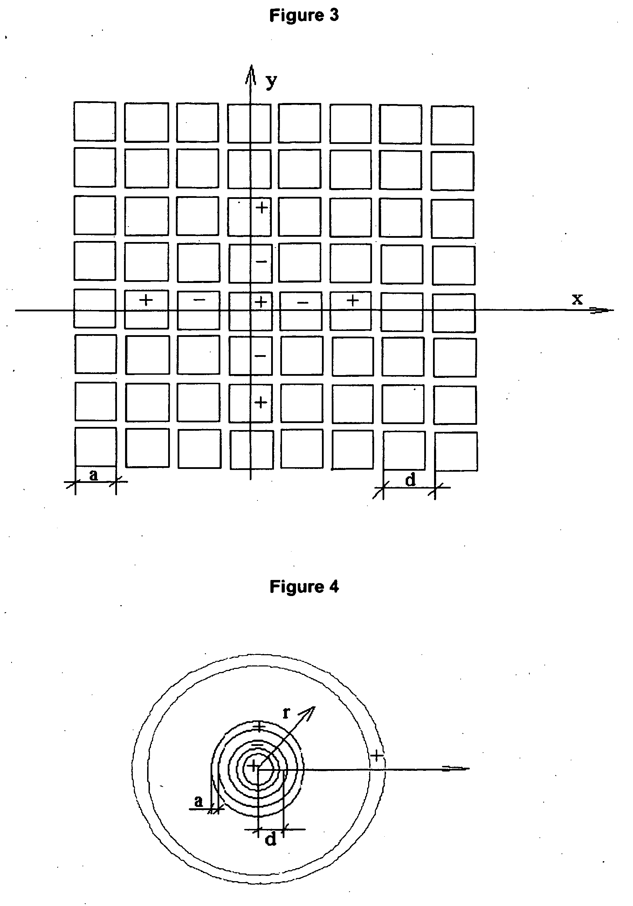

Two possible examples of suitable alternating positive and negative electrode arrangements are shown in Figure 3 and Figure 4. In Figure 3 a matrix (chessboard) arrangement of alternating positive and negative electrodes is demonstrated. Alternatively, a concentric ring arrangement of alternating positive and negative electrodes as shown in Figure 4 could be used in the present invention. In these figures (a) denotes the diameter of the individual electrodes and (d) denotes the repeat distance of the individual electrodes. In Figure 3 (x) and (y) denote the diameter of the matrix electrode arrangement and in Figure 4 (r) denotes the radius of the concentric electrode arrangement.

Such a matrix or concentric arrangement could not be used with the flexible strip applicators of Gelvich and Mazokhin as in their case the bent cylinder axis has to be parallel with the electric field vector.

It is important to note that the alternating arrangement is not limited to only the arrangements shown in Figures 3 and 4, and that other arrangements of alternating positive and negative electrodes would be suitable for use in the present invention. For example, the matrix could be deformed to a Cantor-dust fractal or the concentric rings could be replaced by non-concentrically arranged or by elliptically grouped rings. Also the individual electrode elements could be of equivalent sizes or of non-equivalent sizes, depending on the aim of the treatment. Many different alternating positive and negative electrode arrangements would be suitable for the practice of the present invention. The most important feature of the inventive hyperthermia device is that the positive and negative electrodes on the hyperthermia device applicator are placed in the same plane and that positive and negative electrodes are arranged in an alternating pattern.

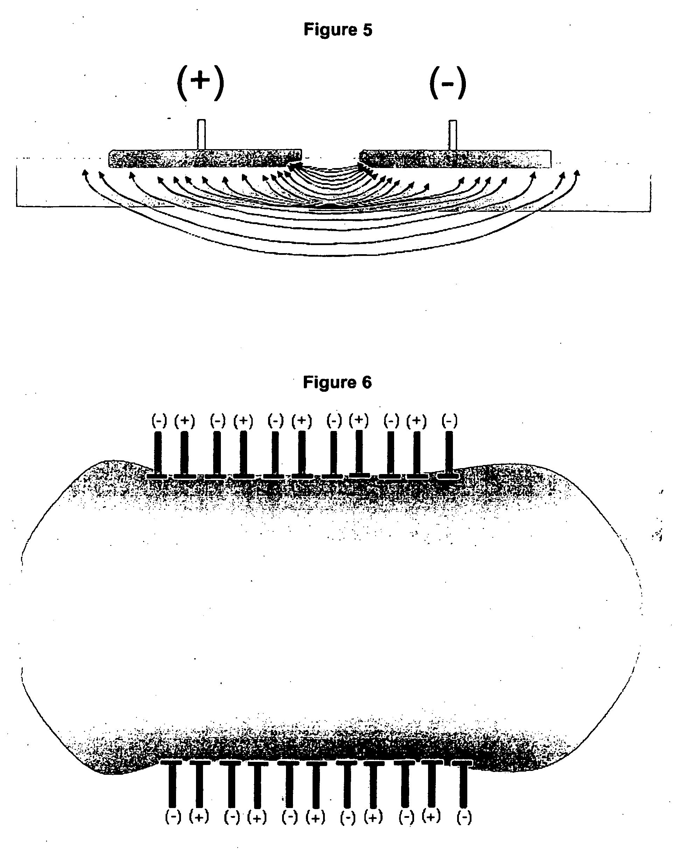

In the electric field capacitive coupled energy transfer means of the present invention the opposing electrodes are present in the same plane and all face the target surface tissue site. As shown in Figure 5, the target tissue connects the electrodes in the same plane and the alternating positive and negative electrodes act as active electrodes and counter electrodes in the same plane. Of course the plane containing the electrodes could also be a flexible plane where the electrodes can be angled toward or away from each other to better fit to uneven surfaces.

The inventive electric field capacitive coupled energy transfer means does not require the use of an opposing counter electrode. However a conventional counter electrode, as described for Figure 1, could be used with the inventive electric field coupled energy transfer means to direct energy to deeper tissues as well as to the surface tissues. Alternatively two opposing electric field capacitive coupled energy transfer means having the inventive arrangement of alternating positive and negative electrodes could be used as the active electrode and the counter electrode as shown in Figure 6.

One simple method of producing the inventive alternating positive and negative electrode arrangements would be to etch the desired alternating positive and negative electrode arrangement onto a conventional printed circuit board. However any construction of the electric field coupled energy transfer means is acceptable if the electric field induced by them enters into the surface tissue and provides homogenous distribution of energy in that tissue.

The individual electrodes of the electric field capacitive coupled energy transfer means can be electrically isolated from one another or alternatively a transmitter bolus could be provided which covers all the electrodes as a group. The alternating electrodes covered by a transmitter bolus will provide a uniform and homogeneous energy field to the target tissue and the Specific Absorption Rate (SAR) will be uniform and homogeneous across the whole contact area between the bolus and the patient surface. In the case of individual electrode isolation the SAR of the tissue between the electrodes will be lower than the SAR of the tissue directly below an electrode. However this variation in SAR will only be significant when the gap between the individual electrodes is large.

The power supply to the electrodes can be symmetrical and continuous, or symmetrical and pulse supplied. Alternatively the power supply to the electrodes can be asymmetric and continuous, or asymmetrical and pulse supplied. The power supply could also be of various frequencies supplied homogeneously to the entire electrode arrangement or supplied at different frequencies to different individual electrodes in the electrode arrangement to provide treatment with parallel impedance control. Thus, each of the multiple electrodes can have an independent frequency. The range of frequencies supplied can be from below detectable the limit (effectively measured as 0 MHz) to 500 MHz. For treatment of surface tissue diseases and conditions the frequency is preferably 13.56 MHz, or 0.5 times, 2 times or 3 times this value (i.e. 6.78 MHz, 27.12 MHz or 40.68 MHz), and for impedance control the frequency is preferably 50 kHz.

The inventive electric field capacitive coupled energy transfer means is useful in the treatment and monitoring of diseases and conditions of surface tissues. These surface tissues comprise the epidermis, dermis and subcutaneous tissue layers and joint tissues.

The epidermis is the upper tissue layer of the skin and the dermis layer occurs immediately below the epidermis. The depth of the epidermis layer is between approximately 0.5 to 1.5 mm and the depth of the dermis layer is between approximately 0.3 to 3.0 mm. The depth of the layers varies between sites, for example, the eyelids have very thin epidermis and dermis layers and the feet have very thick epidermis and dermis layers. The subcutaneous tissue is situated below the dermis layer, and the depth of this layer varies throughout the body and also varies at the same site between individuals.

The joint tissues include fibrous connective tissue and cartilage tissue, including hyaline, elastic, fibrous and articular cartilage. These joint and cartilage tissues which occur in close proximity to the skin surface can also be treated using the inventive electric field coupled energy transfer means.

The inventive electric field capacitive coupled energy transfer means described above can also be used for the measurement of electrical properties of surface tissues. Bioimpedance measurement can be performed simply by using two pairs of electrodes. A current is applied by one electrode pair and detected by a second electrode pair. The change in the voltage detected by the second electrode pair allows the measurement of the impedance of the tested tissue. A decrease in the impedance denotes an increase in blood flow (blood perfusion).

The inventive arrangement comprising multiple electrodes can be used simultaneously to treat surface tissue and to monitor the effect of the treatment. As the inventive arrangement comprises multiple electrodes a number of electrodes in the arrangement can be used as treatment electrodes for treating the surface tissue and a separate number of electrodes in the arrangement can be used as measurement electrodes for monitoring the effect of the treatment. The desired frequencies supplied to the tissue by the treatment electrodes and by the measurement electrodes can be individually set by the operator. The frequency can also be set to be different between individual electrodes within the treatment electrodes or within the measurement electrodes.

For alternating positive and negative electrodes in a matrix arrangement Figure 7 shows the relationship between SAR and penetration depth for a given target material and Figure 8 shows the relationship between voltage and the number of electrode pairs between 1 and 16 for a given target material. For alternating positive and negative electrodes in a concentric ring arrangement Figure 9 shows the relationship between SAR and penetration depth for a given target material and Figure 10 shows the relationship between voltage and the number of electrode pairs between 1 and 16 for a given target material. Figures 11 and 12 compare the above matrix and ring electrode arrangements.

Thus the present invention is directed to an electric field capacitive coupled energy transfer means for directing energy to a target tissue of a mammal, the electric field coupled energy transfer means comprising multiple positive and negative electrodes, wherein the target tissue is a surface tissue, cartilage or joint tissue, and wherein the multiple positive and negative electrodes are arrayed in an alternating positive and negative electrode arrangement. A near field arrangement is required for capacitive coupling. Thus the capacitive coupling device can be a near field capacitor antenna where distance between the energy source and target tissue is much less than the wavelength of the applied RF-wave.

The alternating positive and negative electrode arrangement of the present invention can be a matrix arrangement, as shown in Figure 3.

Alternatively, the alternating positive and negative electrode arrangement of the present invention can be a concentric ring arrangement, as shown in Figure 4.

Specifically the electric field capacitive coupled energy transfer means of the present invention is useful for the treatment of conditions of the surface tissues. The surface tissues include the dermis, epidermis, subcutaneous tissues, cartilage and joint tissues.

The diameter of the individual electrodes in the matrix arrangement is preferably between 1 mm and 10 mm diameter, more preferably between 2 mm and 10 mm diameter, even more preferably between 2 mm and 8 mm diameter and most preferably between 3 mm and 5 mm diameter.

Thus the number of electrode pairs in the matrix arrangement is preferably between 1 and 32, more preferably between 5 and 16, most preferably between 8 and 16.

The diameter of the individual alternating concentric rings is preferably between 1 mm and 10 mm diameter, more preferably between 2 mm and 10 mm diameter, even more preferably between 2 mm and 8 mm diameter and most preferably between 3 mm and 5 mm diameter.

The number of electrode pairs in the concentric ring arrangement is preferably between 1 and 32, more preferably between 5 and 16, most preferably between 8 and 16.

The penetration depth of the electric field coupled energy transfer means is preferably between 1 mm and 10 mm, more preferably between 1 mm and 6 mm, most preferably between 1 mm and 3 mm.

The alternating positive and negative electrode arrangement of the electric field capacitive coupled energy transfer means is preferably provided on a printed circuit board. Such an arrangement can also be flexible.

The electric field capacitive coupled energy transfer means can comprise treatment electrodes for treating the surface tissue and measurement electrodes for monitoring the effect of the treatment.

Further each of the multiple positive and negative electrodes of the electric field capacitive coupled energy transfer means can have an independent frequency.

It is also disclosed the use of the inventive electric field capacitive coupled energy transfer means comprising multiple positive and negative electrodes wherein the multiple positive and negative electrodes arrayed in an alternating positive and negative electrode arrangement to provide an improved method of hyperthermia treatment for the treatment of a target tissue of a mammal. Preferably the target tissue is a surface tissue.

Tumour cells are preferentially sensitive to heat in comparison to normal tissue. Thus, the hyperthermia device of the present invention can be used for treatment and/or prophylaxis of epithelial cancers. All types of cancers that occur in surface tissues can be treated using the inventive device, for example, basal cell carcinoma, squamous cell carcinoma, melanoma, Kaposi sarcoma, cutaneous lymphomas, skin adnexal tumours, soft tissue sarcomas of the dermis, soft tissue sarcomas subcutaneous tissue, Merkel cell carcinoma, dermatofibrosarcoma protuberans (DFSP) and angiosarcoma.

The inventive electric field capacitive coupled energy transfer means can also be used for the treatment and/or prophylaxis of joint and articular diseases and conditions, for example, rheumatoid arthritis, ankylosing spondylitis and lupus.

The inventive electric field capacitive coupled energy transfer means can also be used to enhance surface microcirculation. This is useful to treat fatigue by detoxification of the treated area.

The inventive electric field capacitive coupled energy transfer means can also be used for cosmetic purposes, for example, cellulite treatment, fat reduction and tissue lifting.

The inventive electric field capacitive coupled energy transfer means can also be used for the treatment of muscle injury in humans or animals.

The inventive electric field capacitive coupled energy transfer means can also be used to enhance surface microcirculation and heat surface tissue to prevent muscle spasms and muscle injury in humans or animals.

The inventive electric field capacitive coupled energy transfer means can also be used for the treatment of lipomas in humans or animals.

The inventive electric field capacitive coupled energy transfer means can also be used for the treatment of any other disease or condition that would benefit from the delivery of energy selectively to surface tissue.

The electric field capacitive coupled energy transfer means can also be used in combination with other forms of cancer therapy, for example chemotherapy, radiotherapy and surgery.

For complementing radiotherapy mainly the increased blood perfusion, and as a consequence, the increased oxygenisation results in an improved radiotherapy treatment outcome. To complement surgery it can be applied preoperatively, intraoperatively for coagulation of a capillary-enriched area and/or cancer treatment and postoperatively to avoid the dissemination of the malignant cells.

In particular it is known that hyperthermia treated tumours demonstrate a higher sensitivity to chemotherapy drugs due to increased blood circulation to the tumour and increased cellular metabolism rates in the tumour. Therefore the hyperthermia device of the present invention can be used in combination with chemotherapy treatment with cytostatic and/or cytotoxic drugs. Examples of some cytostatic and/or cytotoxic drugs are actinomycin D, aminoglutethimide, amsacrine, anastrozole, antagonists of purine and pyrimidine bases, anthracycline, aromatase inhibitors, asparaginase, antiestrogens, bexaroten, bleomycin, buselerin, busulfan, camptothecin derivates, capecitabine, carboplatin, carmustin, chlorambucil, cisplatin, cladribin, cyclophosphamide, cytarabin, cytosinarabinoside, alkylating cytostatics, dacarbacine, dactinomycin, daunorubicin, docetaxel, doxorubicin (adriamycin), doxorubicin lipo, epirubicin, estramustine, etoposide, exemestane, fludarabine, fluorouracil, folic acid antagonists, formestane, gemcitabine, glucocorticoids, goselerin, hormones and hormone antagonists, hycamtin, hydroxy urea, idarubicin, ifosfamid, imatinib, irinotecan, letrozole, leuprorelin, lomustine, melphalan, mercaptopurine, methotrexate, miltefosine, mitomycin, mitosis inhibitors, mitoxantron, nimustine, oxaliplatin, paclitaxel, pentostatin, procarbacin, tamoxifen, temozolomide, teniposide, testolactone, thiotepa, tioguanine, topoisomerase inhibitors, topotecan, treosulfan, tretinoin, triptorelin, trofosfamide, vinblastine, vincristine, vindesine, vinorelbine, and antibiotics with cytotoxic activities.

It is also disclosed the use of the inventive electric field capacitive coupled energy transfer means to provide an improved method of monitoring the treatment of surface tissue diseases and conditions.

The electric field capacitive coupled energy transfer means comprising multiple positive and negative electrodes wherein the multiple positive and negative electrodes are arrayed in an alternating positive and negative electrode arrangement can be used for the measurement of electrical properties of a target tissue of a mammal. Preferably the target tissue is a surface tissue.

Further the electric field capacitive coupled energy transfer means comprising multiple positive and negative electrodes wherein the multiple positive and negative electrodes are arrayed in an alternating positive and negative electrode arrangement, can be used for the treatment of a target tissue of a mammal and the simultaneous measurement of electrical properties of the target tissue. Preferably the target tissue is a surface tissue.

The electric field capacitive coupled energy transfer means of the present invention provides the following significant advantages over prior art electric field coupled energy transfer means.

By the use of the inventive electrode arrangement the electric field capacitive coupled energy transfer means energy is selectively delivered to only the surface tissue. For the first time this allows for the effective hyperthermia treatment of surface tissue diseases and conditions while protecting the healthy tissue below from the harmful side effects of tissue heating.

Further the electric field capacitive coupled energy transfer means of the present invention allows impedance selection for the targeted surface regions and impedance control of the same region.

Also the electric field capacitive coupled energy transfer means provides a means for both the treatment of surface tissue and the simultaneous monitoring of the effect of the treatment.

### Description of figures:

- Figure 1: shows an example of a conventional electrical field hyperthermia device with the applicator comprising the active electrode and the transmitter bolus positioned above the target tissue and a counter electrode positioned below the target tissue
- Figure 2: shows the inventive electrode arrangement comprising multiple positive and negative electrodes provided in the same plane
- Figure 3: shows the inventive matrix (chess) arrangement of alternating positive and negative electrodes
- Figure 4: shows the inventive concentric ring arrangement of alternating positive and negative electrodes
- Figure 5: shows the alternating positive and negative electrodes connected in the same plane via the target tissue
- Figure 6: shows the use of two opposing electric field capacitive coupled energy transfer means having the inventive arrangement of alternating positive and negative electrodes

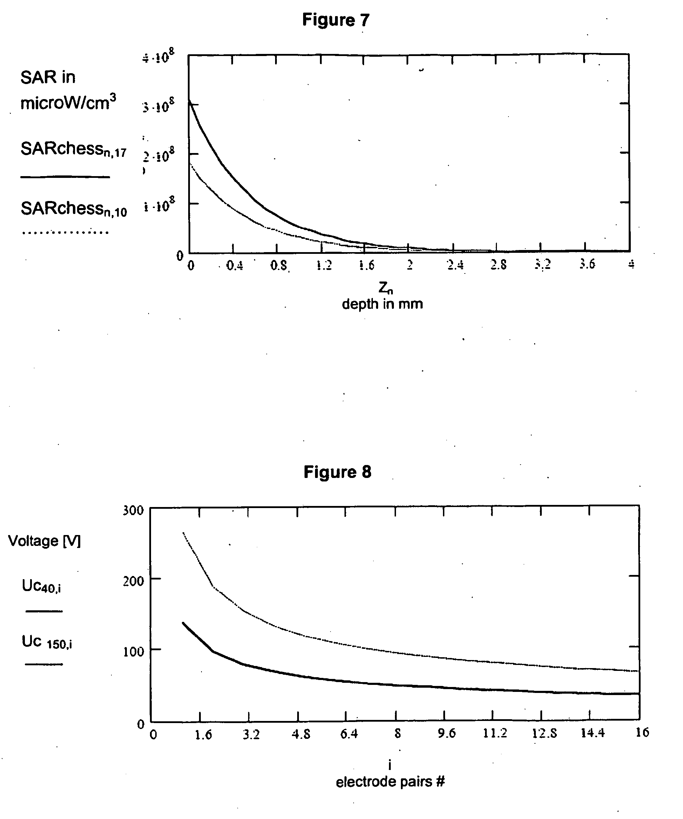

- Figure 7: shows the relationship between SAR and penetration depth for the matrix (chess) arrangement of alternating positive and negative electrodes
- Figure 8: shows the relationship between voltage and number of electrode pairs for the matrix (chess) arrangement of alternating positive and negative electrodes

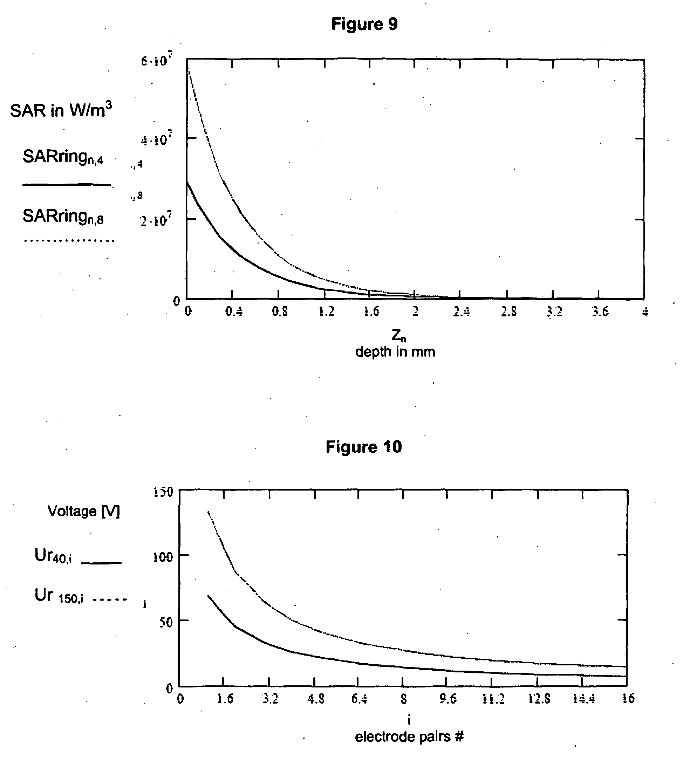

- Figure 9: shows the relationship between SAR and penetration depth for the concentric ring arrangement of alternating positive and negative electrodes
- Figure 10: shows the relationship between voltage and number of electrode pairs for the concentric ring arrangement of alternating positive and negative electrodes

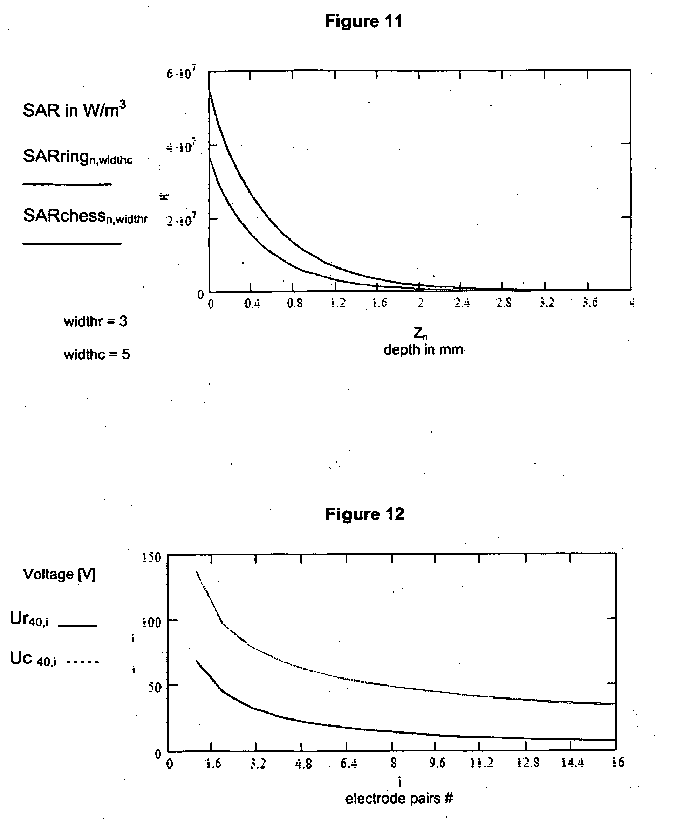

- Figure 11: shows the penetration depth for alternating positive and negative electrode in both a matrix (chess) and concentric ring arrangement
- Figure 12: shows the consistency of voltage delivery for alternating positive and negative electrodes in both a matrix (chess) and concentric ring arrangement.

### EXAMPLE

The relationship between electrode arrangement and the specific heating of surface tissue has been explained above. The following examples model the effect of the inventive electrodes on surface tissue.

In this model the electrical field capacitive coupled energy was provided by the either the inventive matrix electrode arrangement or by the inventive concentric electrode arrangement. For the matrix electrode arrangement and the concentric electrode arrangement the repeat distance (d) of the individual electrodes was set at 5mm. The diameter (a) of the individual electrodes is set at 4 mm for the matrix electrode arrangement and the diameter (a) of the individual electrodes is set at 3 mm for the concentric electrode arrangement. The conductivity of the target material is set at 0.3, the relative permittivity of the target material is set at 20, and the treatment frequency is set at 13.56 MHz.

The results of the modelling calculation for these two electrodes are shown in Figures 7 to 12.

Figure 7 and 8 show the result of the modelling for the inventive matrix (chess) electrode arrangement. Figure 7 shows the relationship between SAR and penetration depth for a given target material and Figure 8 shows the relationship between voltage and the number of electrode pairs between 1 and 16 for a given target material. Figure 7 shows the SAR at an applied power of 10 W and 17 W and demonstrates that the SAR is reduced to close to zero at a depth of more than 3 mm. Figure 8 shows the voltage at applied amplitudes of 40 V and 150 V and demonstrates that the voltage delivered to the tissue remains relatively constant when the number of electrode pairs is between 5 and 16.

For alternating positive and negative electrodes in a concentric ring arrangement Figure 9 shows the relationship between SAR and penetration depth for a given target material and Figure 10 shows the relationship between voltage and the number of electrode pairs between 1 and 16 for a given target material. Figure 9 shows the SAR at applied power of 4 W and 8 W and demonstrates that the SAR is reduced to close to zero at a depth of more than 3 mm. Figure 10 shows the voltage at applied amplitudes of 40 V and 150 V and demonstrates that the voltage delivered to the tissue remains relatively constant when the number of electrode pairs is between 5 and 16.

Figures 11 and 12 show a comparison between the above matrix and ring electrode arrangements. As can be seen in Figure 11 the penetration depth for alternating positive and negative electrode in both a matrix and concentric ring arrangement is limited to less than 3 mm. Figure 12 demonstrates the consistency of voltage delivery for alternating positive and negative electrodes in both a matrix and concentric ring arrangement.

## Claims

1. An electric field capacitive coupled energy transfer means applying radio frequency waves and alternating current for directing energy to a target tissue of a mammal, the electric field coupled energy transfer means comprising multiple positive and negative electrodes,
wherein the target tissue is a surface tissue and
wherein the multiple positive and negative electrodes are arrayed in an alternating positive and negative electrode arrangement.

2. The electric field capacitive coupled energy transfer means of claim 1 wherein the alternating positive and negative electrode arrangement is a matrix arrangement.

3. The electric field capacitive coupled energy transfer means of claim 1 wherein the alternating positive and negative electrode arrangement is a concentric ring arrangement.

4. The electric field capacitive coupled energy transfer means of claim 1 wherein the surface tissue is dermis, epidermis or subcutaneous tissue.

5. The electric field capacitive coupled energy transfer means of claim 1 wherein the surface tissue is a joint or cartilage tissue.

6. The electric field capacitive coupled energy transfer means of claim 2 or 3 wherein the number of electrode pairs is between 2 and 32.

7. The electric field capacitive coupled energy transfer means of claim 2 or 3 wherein the diameter/diagonal of the individual electrodes is between 2 mm and 10 mm.

8. The electric field capacitive coupled energy transfer means of claim 2 or 3 wherein the penetration depth is between 1 mm and 10 mm.

9. The electric field capacitive coupled energy transfer means of claim 1 wherein the alternating positive and negative electrode arrangement is provided on a printed circuit board.

10. The electric field capacitive coupled energy transfer means of claim 1 wherein the multiple positive and negative electrodes comprise treatment electrodes and measurement electrodes.

11. The electric field capacitive coupled energy transfer means of claim 1 wherein the each of the multiple positive and negative electrodes has an independent frequency.

12. The electric field capacitive coupled energy transfer means of claim 1 wherein the multiple positive and negative electrodes are covered by a transmitter bolus.

## Patentansprüche

1. Ein mit einem elektrischen Feld kapazitiv gekoppeltes Mittel für das Übertragen von Energie, das Radiofrequenzwellen und Wechselstrom appliziert, für das Richten von Energie auf ein Zielgewebe eines Säugers, wobei das mit dem elektrischen Feld gekoppelte Mittel für das Übertragen von Energie mehrere positive und negative Elektroden umfasst,
wobei das Zielgewebe ein Oberflächengewebe ist und
wobei die mehreren positiven und negativen Elektroden in einer alternierenden Anordnung von positiven und negativen Elektroden angeordnet sind.

2. Das mit einem elektrischen Feld kapazitiv gekoppelte Mittel für das Übertragen von Energie gemäß Anspruch 1, wobei die alternierende Anordnung von positiven und negativen Elektroden eine Matrixanordnung ist.

3. Das mit einem elektrischen Feld kapazitiv gekoppelte Mittel für das Übertragen von Energie gemäß Anspruch 1, wobei die alternierende Anordnung von positiven und negativen Elektroden eine konzentrische Ringanordnung ist.

4. Das mit einem elektrischen Feld kapazitiv gekoppelte Mittel für das Übertragen von Energie gemäß Anspruch 1, wobei das Oberflächengewebe Dermisgewebe, Epidermisgewebe oder subkutanes Gewebe ist.

5. Das mit einem elektrischen Feld kapazitiv gekoppelte Mittel für das Übertragen von Energie gemäß Anspruch 1, wobei das Oberflächengewebe ein Gelenkgewebe oder ein Knorpelgewebe ist.

6. Das mit einem elektrischen Feld kapazitiv gekoppelte Mittel für das Übertragen von Energie gemäß Anspruch 2 oder 3, wobei die Anzahl der Elektrodenpaare zwischen 2 und 32 liegt.

7. Das mit dem elektrischen Feld kapazitiv gekoppelte Mittel für das Übertragen von Energie gemäß Anspruch 2 oder 3, wobei der Durchmesser/die Diagonale der einzelnen Elektroden zwischen 2 mm und 10 mm liegt.

8. Das mit dem elektrischen Feld kapazitiv gekoppelte Mittel für das Übertragen von Energie gemäß Anspruch 2 oder 3, wobei die Eindringtiefe zwischen 1 mm und 10 mm liegt.

9. Das mit dem elektrischen Feld kapazitiv gekoppelte Mittel für das Übertragen von Energie gemäß Anspruch 1, wobei die alternierende Anordnung von positiven und negativen Elektroden auf einer Schaltplatine bereitgestellt wird.

10. Das mit dem elektrischen Feld kapazitiv gekoppelte Mittel für das Übertragen von Energie gemäß Anspruch 1, wobei die mehreren positiven und negativen Elektroden Behandlungselektroden und Messelektroden umfassen.

11. Das mit dem elektrischen Feld kapazitiv gekoppelte Mittel für das Übertragen von Energie gemäß Anspruch 1, wobei jede der mehreren positiven und negativen Elektroden eine unabhängige Frequenz aufweist.

12. Das mit dem elektrischen Feld kapazitiv gekoppelte Mittel für das Übertragen von Energie gemäß Anspruch 1, wobei die mehreren positiven und negativen Elektroden mit einem Transmitterbolus bedeckt sind.

## Revendications

1. Un moyen couplé capacitivement avec un champ électrique pour la transmission d'énergie, appliquant des ondes radiofréquences et du courant alternatif pour diriger d'énergie sur le tissu cible d'un mammifère, dans lequel le moyen couplé avec un champ électrique pour la transmission d'énergie comprend plusieurs électrodes positives et négatives,
dans lequel le tissu cible est un tissu superficiel et
dans lequel les plusieurs électrodes positives et négatives sont disposées dans un ordre alternant.

2. Le moyen couplé capacitivement avec un champ électrique pour la transmission d'énergie selon la revendication 1, dans lequel l'ordre alternant des plusieurs électrodes positives et négatives est un ordre matrice.

3. Le moyen couplé capacitivement avec un champ électrique pour la transmission d'énergie selon la revendication 1, dans lequel l'ordre alternant des plusieurs électrodes positives et négatives est un ordre de cercles concentriques.

4. Le moyen couplé capacitivement avec un champ électrique pour la transmission d'énergie selon la revendication 1, dans lequel le tissu superficiel est tissu dermatique, épidermatique ou sous-cutané.

5. Le moyen couplé capacitivement avec un champ électrique pour la transmission d'énergie selon la revendication 1, dans lequel le tissu superficiel est un tissu articulaire ou cartilagineux.

6. Le moyen couplé capacitivement avec un champ électrique pour la transmission d'énergie selon les revendications 2 ou 3, dans lequel le nombre des paires d'électrodes est entre 2 et 32.

7. Le moyen couplé capacitivement avec un champ électrique pour la transmission d'énergie selon les revendications 2 ou 3, dans lequel le diamètre / la diagonale des électrodes individuelles est entre 2 mm et 10 mm.

8. Le moyen couplé capacitivement avec un champ électrique pour la transmission d'énergie selon les revendications 2 ou 3, dans lequel la profondeur de pénétration est entre 1 mm et 10 mm.

9. Le moyen couplé capacitivement avec un champ électrique pour la transmission d'énergie selon la revendication 1, dans lequel l'ordre alternant des plusieurs électrodes positives et négatives est fourni sur une platine de commutation.

10. Le moyen couplé capacitivement avec un champ électrique pour la transmission d'énergie selon la revendication 1, dans lequel les plusieurs électrodes positives et négatives comprennent des électrodes de traitement et des électrodes de mesure.

11. Le moyen couplé capacitivement avec un champ électrique pour la transmission d'énergie selon la revendication 1, dans lequel chacune des plusieurs électrodes positives et négatives a une fréquence indépendante.

12. Le moyen couplé capacitivement avec un champ électrique pour la transmission d'énergie selon la revendication 1, dans lequel les plusieurs électrodes positives et négatives sont couvertes avec un bolus transmetteur.
